# EUROPEAN PATENT SPECIFICATION

(11) **EP 2 297 160 B1**
(45) Date of publication and mention of the grant of the patent: **24.09.2014**
(21) Application number: 09765266.3
(22) Date of filing: 19.06.2009
(51) Int. Cl.: C07D 489/02, C07D 221/28

(54) **PROCESS FOR THE SYNTHESIS OF N-DEMETHYLATED MORPHINANE COMPOUNDS**
VERFAHREN FÜR DIE SYNTHESE VON N-DEMETHYLIERTEN MORPHINANVERBINDUNGEN
PROCÉDÉ DE SYNTHÈSE DE COMPOSÉS MORPHINANES N-DÉMÉTHYLÉS

(30) Priority: 20.06.2008 AU 2008903154
(43) Date of publication of application: 23.03.2011
(73) Proprietor: TPI Enterprises Ltd., Cressy, TAS 7302 (AU)
(72) Inventor: BOS, Richard, Launceston Tasmania 7250 (AU); RITCHIE, Jarrod, David, Launceston Tasmania 7250 (AU)
(74) Representative: Fiussello, Francesco
(86) International application number: PCT/AU2009/000786
(87) International publication number: WO 2009/152577

(56) References cited:
- WO-A1-02/16367
- WO-A1-2005/028483
- DONG ET AL.: "New Methodology for the N-Demethylation of Opiate Alkaloids", J. ORG. CHEM., vol. 72, no. 26, 1 December 2007 (2007-12-01), pages 9881-9885, XP55033951, ISSN: 0022-3263, DOI: 10.1021/jo071171q
- THAVANESWARAN ET AL.: "Further investigation of the N-demethylation of tertiary amine alkaloids using the non-classical Polonovski reaction", BIOORG. MED. CHEM. LETT., vol. 16, no. 11, 1 June 2006 (2006-06-01), pages 2868-2871, XP025106136, ISSN: 0960-894X, DOI: 10.1016/J.BMCL.2006.03.017 [retrieved on 2006-06-01]
- THAVANESWARAN ET AL.: "N-Demethylation of Alkaloids", NATURAL PRODUCT COMMUNICATIONS, vol. 1, no. 10, 2006, pages 885-897, XP009161551,
- ROSENAU ET AL.: "A General, Selective, High-Yield N-Demethylation Procedure for Tertiary Amines by Solid Reagents in a Convenient Column Chromatography-like Setup", ORG. LETT., vol. 6, no. 4, 1 February 2004 (2004-02-01), pages 541-544, XP55003263, ISSN: 1523-7060, DOI: 10.1021/ol036319g
- MCCAMLEY, K. ET AL.: 'Efficient N-demethylation of opiate alkaloids using a modified non-classical Polonovski reaction' J. ORG. CHEM. vol. 68, no. 25, 2003, pages 9847 - 9850, XP008138614

## Description

### FIELD

The present invention relates to a process for the preparation of an *N*-demethyl morphinane. In one form the preparation of the *N*-demethyl morphinane involves the *N*-demethylation of an *N*-methyl morphinane. In particular the present process is useful in the preparation *N*-demethyl morphinane by *N*-demethylation of *N-*methyl morphinanes extracted from plants of the genus *Papaver* of the family *Papaveraceae.* An example of a suitable *N*-methyl morphinane that may be usefully subjected to the process of the present invention is thebaine.

### BACKGROUND

There are a large number of *N*-methyl morphinane compounds known to man. Compounds of this type include the opiate alkaloids obtained from the poppy plants of the *Papaveraceae* familly. The specific alkaloids that currently are extracted from the *Papaver* genus which are of commercial value include morphine, codeine, thebaine, and oripavine. The structures of these four alkaloids are shown below.

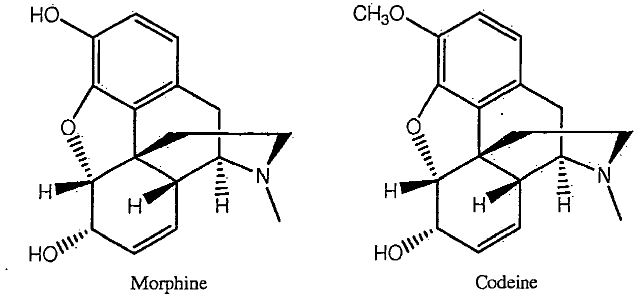

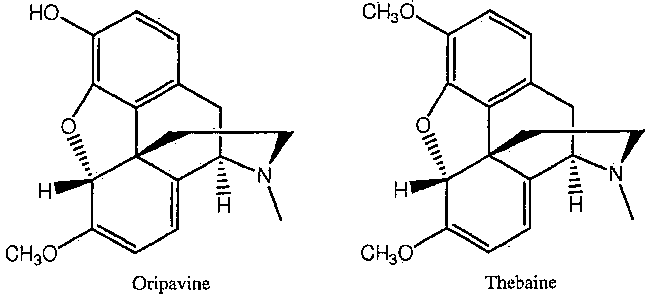

As can be seen from the structures shown above these four compounds possess a high degree of structural similarity. Indeed in classifying structures of this type it is generally accepted that they all contain the morphinane skeleton shown bellow.

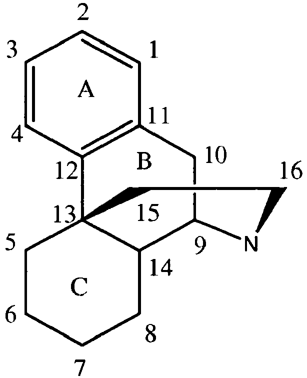

As can be seen typically within the morphinane framework there is an oxygen linkage between the carbons at positions 4 and 5. In addition differences between the various morphinanes are observed in the C ring where different levels of unsaturation are observed.

Whilst many of the naturally occurring *N*-methyl morphinanes have interesting biological activity in their own right there has been significant research conducted to identify improved second generation semi-synthetic morphinanes with improved properties. Much of the work in this area has been focussed on replacement of the methyl group of the *N*-methyl morphinane with other more elaborate side chains with a view to modifying the activity of the morphinane. Accordingly in these processes the important synthetic precursor is the *N-*demethyl morphinane or a protected form thereof which is then further elaborated to the final second generation product.

For example thebaine and oripavine are currently employed as synthetic precursors to a number of opioids including products such as naloxone, naltrexone and buprenorphine. Naltrexone is an opioid receptor antagonist used primarily in the management of alcohol dependence and opioid dependence. Alexon is a drug used to counter the effects of opioid overdose, for example heroin or morphine overdoses. Alexon is specifically used to counteract life-threatening depression of the central nervous system and respiratory system. Buprenorphine is an opioid drug with partial agonist and antagonist actions. Accordingly preparation of the corresponding *N*-demethyl morphinanes (nor thebaine and nor-oripavine respectively) is an important process.

A key step in the conversion of the naturally occurring *N*-methyl morphinanes to second generation products is the *N*-demethylation of the tertiary nitrogen atom in the *N-*methyl morphinane to form the secondary amine (*N*-demethyl morphinane) which can then be further elaborated to the desired second generation product. Accordingly there is a need for the development of processes that provide *N*-demethyl morphinanes especially processes that provide *N*-demethyl morphinanes from the corresponding naturally occurring *N*-methyl morphinanes.

There are a number of methods utilised in the art for a transformation of this type. The transformation has traditionally been carried out using the von Braun reaction which utilises cyanogen bromide as the key reactant. This reaction is undesirable however, due to the toxicity of the reagent which makes it unattractive to use in an industrial setting.

An alternative to the von Braun reaction is the use of chloroformate reagents such as vinyl chloroformate. These reagents typically lead to *N*-demethylation of the *N*-methyl morphinane in high yields and the resulting carbonates are generally able to be readily converted to the desired secondary amine. Unfortunately, however, this process is not industrially desirable as the reagents are very expensive and therefore the ability to scale up the process is severely limited by economic concerns.

In 1985, Monkovic and co-workers reported a general method for the *N* dealkylation of amine oxides using a soluble form of an Fe(II) catalyst reducing agent. A relevant example in this publication involved the dealkylation of a morphinane *N*-oxide. A biphasic solvent system was employed to minimise the undesirable contamination of the dealkylated morphinane product with the iron catalyst reducing agent caused by extensive emulsion formation resulting from iron hydroxides, and related iron compounds. The use of a biphasic system restricts the use of solvents to those that are immiscible with water, which is employed as the solvent for the iron catalyst reducing agent. The quantity of iron catalyst reducing agent used in this work was 40 mole % illustrating the relatively high levels of reducing agent required for reasonable reaction time frames.

Recently Glaxo SmithKline have published a process (WO02/16367) in which the *N-*methyl morphinane is also first oxidised to form the corresponding *N*-oxide which is then also reduced using an iron (II) reducing agent to form the *N*-demethyl morphinane. The difficulties with this mono phasic approach involving soluble forms of iron invariably relates to the complex work up procedures of these reactions on a large scale to isolate and subsequently purify the products to a pharmaceutically acceptable standard. Whilst this can be achieved, it results in a substantial increase in the cost of production and time. Indeed the authors themselves state that "in a preferred embodiment, the reduction step can be affected with a catalytic amount of reducing agent. This is advantageous as an excess of Fe (II) and Fe (III) species can result in handling difficulties by formation of thick emulsions during the reaction work ups". The quantity of iron (II) used in this work was 25 mole % of the starting morphinan-*N*-oxide concentration.

The necessary quantity of Iron (II) catalyst was reported by McCamley et al. ( J.Org.Chem. 2003, 68, 9847-9850), to be at least 50 mole % to ensure a complete reaction. It should also be noted that the work reported by McCamley *et al.,* and that reported in patents WO02/16367 and WO 2005/028483 A1 varied from 25 to 200 mole % catalyst. Given that the level of Iron use was so high, the workup procedures employed in these works were heavily directed towards cleanup of the reacted alkaloid.

As such due to the work up issues attempts have been made to overcome the problem presented by the presence of the reducing agent. One approach has been to reduce the amount of reducing agent employed in order to reduce the amount of reducing agent (such as iron) to be removed during purification. Unfortunately, the use of lower levels of an iron (II) porphyrin catalyst (10 mole % with respect to iron) has also been found by Dong and Scammells (J. Org. Chem., 2007, 72, 9881-9885) to result in incomplete reactions, despite extended reaction times of up to 11 days. This therefore strongly suggests that reduced levels of reducing agent is not practicable on an industrially acceptable time-scale. Further methods for preparing N-demethyl morphinanes are disclosed in Thavaneswaran et al. (Bioorg. Med. Chem. Lett. 2006, 16, 2868-2871 and Natural Product communications 2006, 1, 885-897). A method for N-demethylation of tertiary amines is disclosed in Rosenav et al. (Org. lett. 2004, 4, 541-544).

As such approaches have been based on exploring ways in which the reducing agent and the morphinane can be separated thereby removing the problems encountered by possible contamination and workup of the reaction mixture. One approach has been based on the use of a biphasic system as discussed above but this is not always attractive as it restricts the process to the use of solvents to those that are immiscible with water, which is employed as the solvent for the reducing agent. The range of solvents is therefore severely limited and not necessarily acceptable from an industrial perspective.

Another technique that could be contemplated to solve the problem of the possible contamination of the morphinane with the reducing agent is by the use of an immobilised reducing agent or an immobilised morphinane. A skilled worker in the field would not contemplate that this would be possible to achieve on an industrially acceptable scale due to the results achieved with low levels of the reducing agent in solution. A skilled worker would appreciate that in order to achieve the reaction in an industrially acceptable time frame using the levels of reducing agent shown to be required by both McCamley and the GlaxoSmithKline patents discussed above would require unacceptably high amount of solid support to be used in the process. This is because a skilled addressee would appreciate that there are severe limitations on the amount of reducing agent or morphinane that can be loaded on a solid support. As such in order to run the reactions taught by McCamley on an immobilised material would potentially overcome the issues related to contamination, but would create further issues due to the large amount of solid support required which would create its own issues in relation to reaction work up in an industrial setting.

In addition in many of these processes there is a risk of residual materials being isolated along with the *N*-demethyl morphinane or protected form thereof such as metal species in solution which is clearly undesirable from a pharmaceutical perspective where it is desirable that as many impurities as possible are removed.

Accordingly the known processes for the production of *N*-demethyl morphinanes or protected forms thereof typically involve *N*-demethylation of *N*-methyl morphinanes or protected forms thereof which either involve the use of undesirable and toxic starting materials, expensive reagents or involve reaction work ups that lead to the procedures being undesirable on a large scale and have a risk of residual contamination. As such there is still a need to develop improved processes for preparation of *N*-demethyl morphinanes or protected forms thereof especially processes that involve the *N*-demethylation of naturally occurring *N*-methyl morphinanes as these are the cheapest available starting materials in an industrial sense.

### SUMMARY

The present applicants have now found that *N*-demethyl morphinanes can be readily produced by reduction of the corresponding *N*-methyl morphinane *N*-oxide using an immobilised reducing agent as claimed in claim 1. The present applicants have found that immobilised reducing agents are typically as effective as non-immobilised reducing agents in the reduction of the morphinane *N*-oxides and allow for ease of separation of the reducing agent from the *N*-demethyl morphinane via filtration once the reduction reaction has been conducted. Accordingly once the reduction reaction has proceeded, the immobilised reducing agent is typically simply filtered off. The advantages to this approach are that it dramatically simplifies the separation of the desired product from the reducing agent and minimises the possibility of the final product containing residual reducing agent which is desirable from a pharmaceutical perspective.

In one aspect the present invention provides a process for the preparation of an *N-*demethyl morphinane the process including:
(a) providing an *N*-methyl morphinane *N*-oxide or a protected form thereof;
(b) reacting the *N*-methyl morphinane *N*-oxide or a protected form thereof with an immobilised reducing agent under reducing conditions in a reduction reaction to form the *N-*demethyl morphinane.

The process of the present invention may theoretically be carried out on any *N-*methyl morphinane *N*-oxide. In one form providing an *N*-methyl morphinane *N*-oxide includes reacting an *N*-methyl morphinane with an oxidising agent under oxidising conditions in an oxidation reaction to form the *N*-methyl morphinane *N*-oxide.

In this form any suitable *N*-methyl morphinane may be used. In one embodiment the *N*-methyl morphinane is an alkaloid extracted from plants of the genus *Papaver* of the family *Papaveraceae.* In one embodiment the *N*-methyl morphinane is selected from the group consisting of morphine, oripavine, codeine, thebaine, and heroin or a protected form thereof. In one specific embodiment the *N*-methyl morphinane is thebaine or a protected form thereof. In another specific embodiment the *N-*methyl morphinane is oripavine or a protected form thereof.

If the process utilises an oxidising step the oxidising agent used in oxidation reaction may be any suitable oxidising agent. In one embodiment the oxidising agent is selected from the group consisting of hydrogen peroxide, m-chloroperbenzoic acid and peracetic acid. In addition any suitable amount of oxidising agent may be used to effect the conversion. In one embodiment a molar excess of oxidising agent is used in the oxidation reaction.

The immobilised reducing agent is a metal ion bound to a solid support. A wide variety of reducing metal ions may be used, for example, vanadium, chromium, manganese, iron, cobalt and indium. In one embodiment the metal ion is Fe(II), in another embodiment the metal ion is V(II). An advantage of the use of an immobilised reducing agent is that it allows the use of toxic reducing agents such as vanadium that would not be contemplated in solution synthesis in a pharmaceutical process due to contamination concerns.

The metal ion may be bound to the solid support in any way known in the art. In one embodiment the metal ion is bound to the solid support by being complexed to a ligand which is attached to the solid support. Any suitable ligand for the metal ion may be utilised. In one embodiment the ligand is a nitrogen containing moiety. An example of a suitable nitrogen containing ligand is 2,2'-bipyridyl or a derivative thereof.

The ligand may be directly attached to the solid support or may be bound to the solid support via a linker. In one embodiment the ligand is attached to the solid support via a linker. A wide variety of linkers may be used with the identity of the linker typically being determined by the identity of the solid support, the identity of the ligand being used and the available reagents. In one embodiment the linker has the formula: 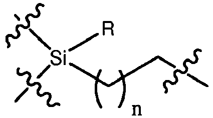 wherein:
R is selected from the group consisting of H, methyl, ethyl, propyl, OCH₃, and OCH₂CH₃;
n is an integer selected from the group consisting of 0, 1, 2, and 3;
and further wherein the Si moiety is attached to the solid support and the CH₂ moiety is attached to the ligand.

In a specific embodiment the linker is a group of the formula:

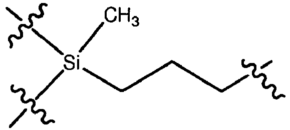

Any of a wide range of solid supports may be utilised in the process of the invention. In one embodiment the solid support is silica particles. In a specific embodiment the silica particles have a particle size of 100 to 700 µm.

The reduction reaction utilised in the process of the present invention may be carried out within a wide range of reaction stoichiometries and under a wide range of reaction conditions. In one embodiment the amount of reducing agent employed is from 0.1 Mole % to 150 Mole %. In one embodiment the amount of reducing agent employed is from 0.1 Mole % to 125 Mole %. In one embodiment the amount of reducing agent employed is from 0.1 Mole % to 100 Mole %. In one embodiment the amount of reducing agent employed is from 0.01 Mole % to 50 Mole %. In another embodiment the amount of reducing agent employed is from 0.1 Mole % to 20 Mole %. In one embodiment the amount of reducing agent employed is from 0.1 Mole % to 10 Mole %. In one embodiment the amount of reducing agent employed is from 1.0 Mole % to 5.0 Mole %.

The reaction may be carried out in any suitable solvent that does not interfere with the reduction reaction. In one embodiment the reaction is carried out in a solvent selected from the group consisting of acetonitrile, ethyl acetate, toluene, ethylbenzene, o-, m- and p-xylene, isopropylbenzene, methylnaphthalene, chlorobenzene, o- and m-dichlorobenzene, o-, m- and p-chlorotoluene,1,2,4-trichlorobenzene, ethyl propyl ether, methyl tert.-butyl ether, n-butyl ethyl ether, di-n-butyl ether, diisobutyl ether, diisoamyl ether, diisopropyl ether, anisole, phenetole, cyclohexyl methyl ether, diethyl ether, ethylene glycol dimethyl ether, tetrahydrofuran, dioxane, heptane, nonane, cyclohexane, methylcyclohexane, decalin, petroleum ether, hexane, light naphtha, 2,2,4-trimethylpentane; 2,2,3-trimethylpentane, 2,3,3-trimethylpentane and octane, methanol, ethanol, propanol, isopropanol, butanol, pentanol, hexanol, water and mixtures thereof. In one specific embodiment the reaction is carried out in a solvent selected from the group consisting of acetonitrile, ethyl acetate, methanol, ethanol, isopropanol and water.

The reduction reaction may be carried out over a wide range of reaction temperatures with it typically being observed that the speed of reaction increases with increasing temperature. In one embodiment the reaction is carried out at a temperature in the range of from 10°C to 125°C. In another embodiment the reduction reaction is carried out at a temperature in the range of from 10°C to 120°C. In one embodiment the reduction reaction is carried out at a temperature in the range of from 50°C to 100°C. In another embodiment the reaction is carried out at a temperature in the range of from 40°C to 90°C. In another embodiment the reduction reaction is carried out at a temperature in the range of from 40°C to 60°C. In another embodiment the reaction is carried out at a temperature of at least 50°C.

The reduction reaction is typically conducted for a period of time necessary to effect the desired level of *N*-demethylation of the *N*-methyl morphinane *N*-oxide. The level of conversion of the *N*-methyl morphinane *N*-oxide to the *N*-demethyl morphinane may be monitored using any of a number of techniques such as TLC and HPLC. Nevertheless in one embodiment the reduction reaction is carried out for a period of from 15 minutes to 48 hours. In one embodiment the reduction reaction is carried out for a period of from 15 minutes to 4 hours. In another embodiment the reaction is carried out for a period of from 1 to 48 hours. In another embodiment the reaction is carried out for from 12 to 24 hours. In another embodiment the reduction reaction is carried out for a period of from 30 minutes to 6 hours.

In order to isolate the final. *N*-methyl morphinane it is typically found that following the reduction reaction the reaction mixture is filtered to separate the *N*-demethyl morphinane from the immobilised reducing agent. This can be carried out using any suitable filtration technology known in the art.

### DETAILED DESCRIPTION

The applicants have found that the process of the present invention is useful for the preparation of a wide range of *N*-demethyl morphinanes especially those prepared from the naturally occurring *N*-methyl morphinanes.

The process of the present invention involves, as a first step, the provision of an *N-*methyl morphinane *N*-oxide. This may be provided synthetically, purchased from another source or provided by oxidation of an *N*-methyl morphinane sourced either synthetically or from nature. The provided *N*-methyl morphinane *N*-oxide may be any suitable morphinane and may be any suitable purity. In general the provision of the *N*-methyl morphinane *N*-oxide involves reacting an *N*-methyl morphinane with an oxidising agent under oxidising conditions in an oxidation reaction to form the *N*-methyl morphinane *N*-oxide.

The process is applicable to the *N*-demethylation of a wide range of *N*-methyl morphinane compounds. In essence the process may be used for the *N*-demethylation of any *N*-methyl morphinane to produce the corresponding *N*-demethyl morphinane. The present applicants have found, however, that the process is particularly applicable to the *N-*demethylation of *N*-methyl morphinanes extracted from plants of the genus *Papaver* of the family *Papaveraceae.* Examples of *N*-methyl morphinanes of this type include morphine, codeine, thebaine and oripavine. The process is particularly applicable to thebaine and oripavine.

In respect of some of the *N*-methyl morphinanes known the functional groups present mean that the *N*-methyl morphinane *per se* is not applicable to the oxidation/reduction cycle in its native state as the functional groups would be affected by the reagents used. In respect of *N*-methyl morphinanes of this type a skilled addressee would be aware that it maybe desirable to first convert the *N*-methyl morphinane to a protected form thereof prior to subjection to the process of the invention. A skilled addressee would be able to readily determine when a protecting group was required and, in addition would be readily able to determine a suitable protective group and the conditions under which it can be added to the N-methyl morphinane to form the protected form thereof.

In general the *N*-methyl morphinane used in the process of the present invention may be of any suitable purity. As such the *N*-methyl morphinane may be pure or may be in the form of a crude extract either from the plant species that the *N*-methyl morphinane was isolated from or from a synthetic source. In general it is found to be useful if the *N-*methyl morphinane is relatively pure as control of the reaction stoichiometry is easier if the purity of the *N*-methyl morphinane to be de-methylated has been established.

The *N*-methyl morphinane is typically provided as a solution in a suitable solvent and this may be done by dissolution of the *N*-methyl morphinane to be *N*-demethylated in the solvent chosen to form a reaction mixture. There are a wide range of suitable solvents including, for example, water, lower alkanols, e.g. ethanol and the like, hydrocarbons, e.g. toluene, ketones, e.g. 2-butanone, halogenated hydrocarbons, e.g. dichloromethane, and mixtures of such solvents. The concentration of the *N*-methyl morphinane may be any suitable concentration although it is typically chosen at a level to minimise solvent use.

This is then typically followed by addition of an oxidising agent to the reaction mixture to convert the *N*-methyl morphinane to the corresponding *N*-Methyl morphinane *N*-oxide. The oxidation reaction may be conducted under a wide range of oxidising conditions using a wide range of oxidants known in the art for the conversion of this type. The oxidation reaction may be carried out by reacting the *N-*methyl morphinane with an appropriate organic or inorganic peroxide. Appropriate inorganic peroxides include for example, hydrogen peroxide, alkali metal or earth alkaline metal peroxides, e.g. sodium peroxide, potassium peroxide; appropriate organic peroxides may comprise peroxy acids such as, for example, benzenecarboperoxoic acid, halo substituted benzenecarboperoxoic acid, e.g. metachloroperbenzoic acid, peroxoalkanoic acids, e.g. peroxoacetic acid and alkylhydroperoxides, e.g. tert-butyl hydroperoxide.

Examples of suitable oxidising agents include metachloroperbenzoic acid, hydrogen peroxide and performic acid and peracetic acid.

The oxidation may be carried out using a wide range of reaction stoichiometries. A suitable reaction stoichiometry is 1.1 to 10 mole excess of oxidising agent. The oxidation reaction may be carried out for any suitable period although a typical time for an oxidation reaction is 16 hours at room temperature. The oxidation reaction may be monitored by any monitoring technique known to determine when the desired level of conversion of the *N-*methyl morphinane to the corresponding *N*-oXide has occurred.

Once formed the *N*-oxide morphinane may be purified or it may be used in the reduction stage of the process as a crude isolate.

The reduction reaction utilised in the process of fhe present invention may be carried out within a wide range of reaction stoichiometries with the exact stoichiometry being selected depending on a number of process variables.

The reduction of the morphinane-*N*-oxide may be achieved with a reaction stoichiometry of from 0.01 mole of reducing agent to 10 mole percent, typically 0.5 mole %. In one embodiment the amount of reducing agent employed is from 0.1 Mole % to 150 Mole %. In one embodiment the amount of reducing agent employed is from 0.1 Mole % to 125 Mole %. In one embodiment the amount of reducing agent employed is from 0.1 Mole % to 100 Mole %. In one embodiment the amount of reducing agent employed is from 0.01 Mole % to 50 Mole %. In another embodiment the amount of reducing agent employed is from 0.1 Mole % to 20 Mole %. In one embodiment the amount of reducing agent employed is from 0.1 Mole % to 10 Mole %. In one embodiment the amount of reducing agent employed is from 1.0 Mole % to 5.0 Mole %. In general the selection of the exact molar amount will depend upon the other reaction conditions and a skilled worker in the art can easily determine the appropriate reaction stoichiometry to use for any particular set of conditions.

The reducing agent may be any suitable reducing agent known in the art that can be immobilised. In one embodiment the reducing agent is a metal ion. A wide variety of reducing metal ions may be used, for example, vanadium, chromium, manganese, iron, cobalt and indium. In one embodiment the metal ion is Fe(II), in another embodiment the metal ion is V(II).

The reduction reaction may be carried out in a suitable solvent or it may be carried out in the absence of a solvent although, in general the presence of a solvent is desirable. In general any suitable solvent may be used with the only requirement being that the solvent does not interfere with the reduction reaction. A large number of suitable solvents exist that fit this criteria with suitable examples of toluene, ethylbenzene, o-, m- and p-xylene, isopropylbenzene, methylnaphthalene, chlorobenzene, o- and m-dichlorobenzene, o-, m-and p-chlorotoluene,1,2,4-trichlorobenzene, ethyl propyl ether, methyl tert.-butyl ether, n-butyl ethyl ether, di-n-butyl ether, diisobutyl ether, diisoamyl ether, diisopropyl ether, anisole, phenetole, cyclohexyl methyl ether, diethyl ether, ethylene glycol dimethyl ether, tetrahydrofuran, dioxane, heptane, nonane, cyclohexane, methylcyclohexane, decalin, petroleum ether, hexane, light naphtha, 2,2,4-trimethylpentane, 2,2,3-trimethylpentane, 2,3,3-trimethylpentane and octane, methanol, ethanol, propanol, isopropanol, butanol, pentanol, hexanol, water and mixtures thereof. The identity of the solvent used in any particular instance will depend upon a number of factors such as the availability of suitable solvents and the N-oxide of the morphinane being used. For example the solvent is typically chosen in which the particular morphinane *N*-oxide has good solubility properties as it is typically found that the more concentrated the morphinane N-oxide is in solution the more economically desirable the process becomes as higher throughputs may be achieved with a smaller capital equipment outlay.

The reduction reaction may be carried out in the presence of a number of other additives such as buffers and the like. By way of example, the applicants have found that the addition of ammonium acetate (1, 2 and 5 molar equivalents relative to the alkaloid input) was also found to favourably enhance the ratio of demethylated alkaloid compared to unconverted alkaloid.

The reaction may be carried out over a wide range of reaction temperatures with it typically being observed that the speed of reaction increases with increasing temperature. Accordingly it is typical that the reaction is carried out at a temperature greater than 40°C. In one embodiment the reaction is carried out at a temperature in the range of from 10°C to 125°C. In another embodiment the reduction reaction is carried out at a temperature in the range of from 10°C to 120°C. In one embodiment the reduction reaction is carried out at a temperature in the range of from 50°C to 100°C. In another embodiment the reaction is carried out at a temperature in the range of from 40°C to 90°C. In another embodiment the reduction reaction is carried out at a temperature in the range of from 40°C to 60°C. In another embodiment the reaction is carried out at a temperature of at least 50°C.

The reduction reaction is typically conducted for a period of time necessary to affect *N*-demethylation of a suitable amount of the *N*-methyl morphinane *N*-oxide. The degree of conversion of *N*-oxide of the *N*-methyl morphinane to the *N*-demethyl morphinane may be monitored using any of a number of techniques such as TLC and HPLC. Nevertheless in one embodiment the reaction is carried out for a period of from 15 minutes to 48 hours. In one embodiment the reduction reaction is carried out for a period of from 15 minutes to 4 hours. In another embodiment the reaction is carried out for a period of from 1 hour to 48 hours. In another embodiment the reaction is carried out for a period of from 12 hours to 48 hours. In another embodiment the reaction is carried out for a period of from 30 minutes to 6 hours.

As will be appreciated by a skilled worker in the field as the *N*-demethyl morphinane and the *N*-methyl morphinane *N*-oxide are readily separated in many instances it is more efficient to terminate the reaction at partial conversion and to then re-subject any isolated *N*-methyl morphinane N-oxide to the process to provide further amounts of the *N*-demethyl morphinane.

The reduction may be carried out with any reducing agent immobilised to a solid support. As used herein the term "immobilised" means that the reducing agent is attached either via a covalent bond or through electrostatic interactions to an inert material which is insoluble in the reaction medium. The reducing agent may be directly attached to the solid support or it may be attached to the solid support via a linker moiety. The solid support may be a noble metal. Suitable noble metals include silver, gold, platinum and palladium. In addition, graphite-based materials, TiO₂, IrO₂, SnO₂, Si-based surfaces or clays may also be used as a solid support. The solid support may be a chip having a surface that is formed from one of the aforementioned materials. The chip itself may be formed from any suitable material including but not limited to glass, plastic or ceramic material. A particularly useful solid support is a silica particle. In another embodiment the solid support is cross-linked polystyrene.

A particularly suitable reducing agent immobilised to a solid support is a metal ion bound to a solid support. It is found that a particularly suitable reducing agent is a metal ion complexed to a ligand which is in turn bound to a solid support via a linker. In principle any reducing agent attached to a solid support via a ligand may be used in the process of the invention. Any suitable ligand moiety may be used with suitable ligands for binding metallic reducing agents being well known in the art. A skilled addressee will typically be able to select a suitable ligand once the identity of the metallic reducing agent has been established.

In essence any ligand that either is, or can be modified to be bound to a solid support may be utilised. Merely by way of example a list of potential ligands are those selected from the group consisting of ethylenediamine, 2,2'-bipyridine, 1,10-phananthroline, acetylacetonate, 1,2-Bis(diphenylphosphino)methane, a corrole, a crown ether, a cryptate, cyclopentadienyl, diethylenetriamine, dimethylglyoximate, ethylenediaminetetraacetate, ethlenediaminetriacetate, glycinate, a porphyrin, 2,2',5',2-terpyridine, triazacyclononane, triethylenetetramine, tris(2-aminoethyl)amine and tris (2-diphenylphosphineethyl)amine.

In certain embodiments the ligand is a nitrogen containing moiety. In one specific embodiment the ligand is 2,2'-bipyridine or a derivative thereof.

A skilled addressee in the art would readily be able to obtain ligands of this type and would be aware of modifications in order to immobilise the ligand onto a solid support either through direct attachment or through binding of the ligand to the solid support through a linker. Nevertheless for the convenience of the reader the synthesis of a suitable immobilised reducing agent will now be discussed. The procedure is generally shown in Scheme 1.

4,4'-Dimethyl-2,2'-bipyridine is reacted with 1 equivalent of lithium diisopropylamine and the anion quenched with allyl bromide to form the functionalised bipyridyl moiety. Hydrosilation of the double bond is then achieved by reaction of the bipyridyl with hydrogen hexachloroplatinate followed by dichloromethylsilane. The reaction product was not isolated but instead silica (dried) was added to the reaction mixture, and gently agitated in the presence of 2.5 equivalents of imidazole to form the ligand immobilised on the silica. The metal to be immobilised is then typically dissolved in a 1 + 1 water ethanol mixture and reacted with the immobilised ligand to form the immobilised metal complex. Additional ligands can then be added to the un-coordinated metal binding sites. Suitable types were oxalate, ethylene diamine, acetylacetonate and 2,2'-bipyridyl.

Whilst the procedure above is detailed for immobilisation of the 4,4'-Dimethyl-2,2'-bipyridine ligand suitable modifications could be made by a skilled addressee for a number of other ligands.

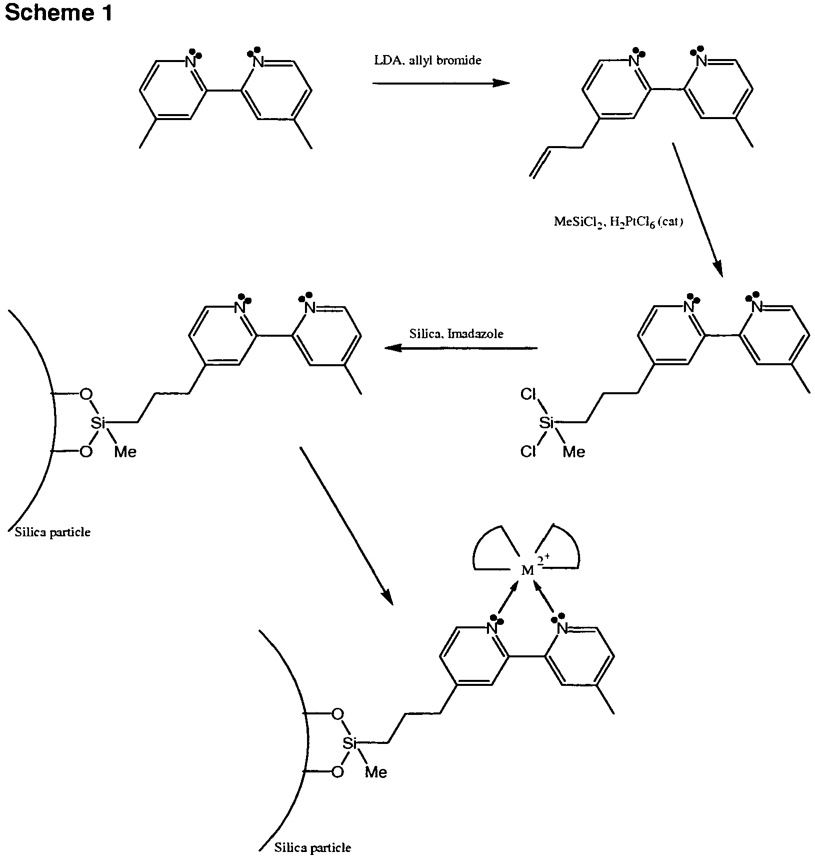

The methods of the invention will now be exemplified with reference to the following examples.

### EXAMPLES

### Example 1 Synthesis of Immobilised Metal reducing agent

1. 4,4'-Dimethyl-2,2'-bipyridine (10.0 grams) was reacted with 1 equivalent of lithium diisopropylamine in dry tetrahydrofuran (250 ml) at -78 °C for a period of 30 minutes. Allyl bromide (1 equivalent) was then added, and the reaction mixture stirred for a further hour. The solution was allowed to warm to room temperature, 10 ml of water was then added to the reaction mixture, prior to removal of the solvent using a rotary evaporator. The resultant viscous material was extracted 3 times with diethylether (3 × 50 ml), the combined extracts were then dried using anhydrous sodium sulfate, filtered and then rotary evaporated to yield 4-(3-butenyl)-4'-methyl-2,2'-bipyridyl, a yellow oil. Yield 95 %.
2. The reaction (a hydrosilation) was performed according to a procedure reported by Sandoval and Pasek, briefly, 4-(3-butenyl)-4'-methyl-2,2'-bipyridyl (10 grams) was placed into dried toluene, to which was added hydrogen hexachloroplatinate (2mg in 3 ml of dried isopropanol), the solution was stirred for a period of five minutes prior to the addition of 4.7 ml of dichloromethylsilane. The reaction then heated to approximately 70°C and stirred overnight. The product of this reaction step was not isolated. Silica (dried) was added to the reaction mixture, and gently agitated in the presence of 2.5 equivalents of imidazole for a period of 12 hours. The now functionalised silica is washed with toluene, dichloromethane, tetrahydrofuran, dimethylformamide, dimethylsulfoxide and water, dilute (1%) sodium hydroxide, water, the process was then reversed, finishing with dichloromethane, and air drying.
3. The metal to be immobilised is dissolved in a 1 + 1 water ethanol mixture and reacted with the immobilised ligand.
4. The immobilised metal is then further reacted with additional ligands. For example oxalate in the form of sodium oxalate or ethylene diamine or other suitable ligands such as acetylacetonate or 2,2'-bipyridyl.
5. The immobilised catalyst is then prepared prior to use by washing with a dilute solution of ascorbic acid (dissolved in a 1+1 water methanol mixture) or other suitable reducing agent to ensure that the metal is in the appropriate oxidation state.

### Example 2

Thebaine-*N*-oxide (210 mg, 6.4 × 10⁻⁴ mol) was dissolved in 20 ml of methanol and added to 5 grams of the Iron functionalised silica (8.1 × 10⁻⁴ mol Iron) made using the procedure outlined in example 1. The % mol ratio of catalyst to analyte was 127 %. The reaction mixture was agitated and heated to 50 °C for 24 hours. The reaction mixture was then cooled and filtered. The catalyst was then washed with additional methanol. The combined liquor and wash methanol solutions were then evaporated to dryness, and assayed by HPLC-UV and NMR spectroscopy. The recovered mass of alkaloid was 195 mg, which comprised of 65 % of nor thebaine and 35 % thebaine.

### Example 3

Thebaine-N-oxide (50 mg, 1.5 × 10⁻⁴ mol) was dissolved in 5 ml of acetonitrile and added to 1 gram of the Iron functionalised silica (2.0 × 10⁻⁴ mol Iron) made using the procedure outlined in example 1. The % mol ratio of catalyst to analyte was 125 %.The reaction mixture was stirred and heated to 75 °C for 2 hours. The reaction mixture was then cooled and filtered and assayed by HPLC-UV. The reaction product comprised of 50 % of nor thebaine and 42 % thebaine, 8 % of thebaine-*N*-oxide remained unreacted.

### Example 4

Thebaine-*N*-oxide (50 mg, 1.5 × 10⁻⁴ mol) was dissolved in 5 ml of ethyl acetate and added to 1 gram of the Iron functionalised silica (2.0 × 10⁻⁴ mol Iron) made using the procedure outlined in example 1. The % mol ratio of catalyst to analyte was 125 %. The reaction mixture was stirred and heated to 75 °C for 2.5 hours. The reaction mixture was then cooled and filtered and assayed by HPLC-UV. The reaction product comprised of 68 % of nor thebaine and 22 % thebaine, 10 % of thebaine-N-oxide remained unreacted.

### Example 5

Thebaine-*N*-oxide (50 mg, 1.5 × 10⁻⁴ mol) was dissolved in 5 ml of ethanol and added to 1 gram of the Iron functionalised silica (2.0 × 10⁻⁴ mol Iron) made using the procedure outlined in example 1. The % mol ratio of catalyst to analyte was 125 %. The reaction mixture was stirred and heated to 75 °C for 2.5 hours. The reaction mixture was then cooled and filtered and assayed by HPLC-UV. The reaction product comprised of 69 % of nor thebaine and 31 % thebaine.

### Example 6

Thebaine-*N*-oxide (500 mg, 1.5 × 10⁻³ mol) was dissolved in 12.5 ml of ethanol and added to 1 gram of a vanadium functionalised silica (2.0 × 10⁻⁴ mol vanadium) made using the procedure outlined in example 1. The % mol ratio of catalyst to analyte was 13.3 %. The reaction mixture was stirred and heated to 125 °C for 15 minutes. The reaction mixture was then cooled and filtered and assayed by HPLC-UV. The reaction product comprised of 38 % of nor thebaine and 62 % thebaine.

### Example 7

Thebaine-*N*-oxide (500 mg, 1.5 × 10⁻³ mol) was dissolved in 12.5 ml of ethanol and added to 1 gram of the Iron functionalised silica (2.0 × 10⁻⁴ mol Iron) made using the procedure outlined in example 1. The % mol ratio of catalyst to analyte was 13.3 %. The reaction mixture was stirred and heated to 125 °C for 15 minutes. The reaction mixture was then cooled and filtered. The catalyst was then washed with additional ethanol. The combined liquor and wash ethanol solutions were then evaporated to dryness, and assayed by HPLC-UV. The reaction product comprised of 38 % of nor thebaine and 62 % thebaine.

### Example 8

Thebaine-N-oxide (500 mg, 1.5 × 10⁻³ mol) was dissolved in 5 ml of water and added to 50 mg of the vanadium functionalised silica (1.0 × 10⁻⁵ mol vanadium) made using the procedure outlined in example 1. The % mol ratio of catalyst to analyte was 0.67 %. The reaction mixture was stirred and heated to 100 °C for 16 hours. The reaction mixture was then cooled and filtered and assayed by HPLC-UV. The reaction product comprised of 19 % of nor thebaine and 72 % thebaine, 9 % of nor thebaine remained unreacted.

### Example 9

Thebaine-*N*-oxide (500 mg, 1.5 × 10⁻³ mol) was dissolved in 5 ml of n-butanol and added to 500 mg of the vanadium functionalised silica (1.0 × 10⁻⁴ mol vanadium) made using the procedure outlined in example 1. The % mol ratio of catalyst to analyte was 6.7 %. The reaction mixture was stirred and heated to 70 °C for 90 minutes. The reaction mixture was then cooled and filtered and assayed by HPLC-UV. The reaction product comprised of 45 % of nor thebaine and 55 % thebaine.

### Example 10

Oxycodone-*N*-oxide (500 mg, 1.5 × 10⁻³ mol) was dissolved in 10 ml of ethanol and added to 500 mg of the vanadium functionalised silica ((1.0 × 10⁻⁴ mol vanadium) with acetylacetonate ligand) made using the procedure outlined in example 1. The % mol ratio of catalyst to analyte was 6.7 %. The reaction mixture was stirred and heated to 70 °C for 180 minutes. The reaction mixture was then cooled and filtered, washed with ethanol, concentrated in vacuo and assayed by HPLC-UV. The reaction product comprised of 65 % of nor oxycodone and 35 % oxycodone, all the starting material had been reacted.

### Example 11

Thebaine-*N*-oxide (200 mg, 6.1 × 10⁻⁴ mol) was dissolved in 5 ml of ethanol and added to 200 mg of the vanadium functionalised silica ((0.5 × 10⁻⁴ mol vanadium) with oxalate ligand) made using the procedure outlined in example 1. The % mol ratio of catalyst to analyte was 8.2 %. Also added to the mixture was ammonium acetate (1 molar equivalent). The reaction mixture was stirred and heated to 90 °C for 45 minutes. The reaction mixture was then cooled and filtered, washed with ethanol, concentrated in vacuo and assayed by HPLC-UV. The reaction product comprised of 50 % of nor thebaine and 50 % thebaine, all the starting material had been reacted. A comparison with the same reaction performed under identical conditions without the addition of ammonium acetate gave reaction mixture ratios of 39 % of nor thebaine and 61 % thebaine, clearly showing that the use of ammonium acetate resulted in an increase in the relative production of the desirable nor product.

Finally, it will be appreciated that various modifications and variations of the methods of the invention described herein will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention that are apparent to those skilled in the art are intended to be within the scope of the present invention.

## Claims

1. A process for the preparation of an *N*-demethyl morphinane, the process including:
(a) providing an *N*-methyl morphinane *N*-oxide;
(b) reacting the *N*-methyl morphinane *N*-oxide with an immobilised reducing agent under reducing conditions in a reduction reaction to form the *N*-demethyl morphinane wherein the immobilised reducing agent is a metal ion bound to a solid support.

2. A process according to claim 1 wherein providing an *N*-methyl morphinane *N*-oxide includes reacting an *N*-methyl morphinane with an oxidising agent under oxidising conditions in an oxidation reaction to form the *N*-methyl morphinane *N*-oxide.

3. A process according to claim 2 wherein the *N*-methyl morphinane is selected from the group consisting of morphine, oripavine, codeine, thebaine and heroin.

4. A process according to claim 2 or 3 wherein the oxidising agent is selected from the group consisting of hydrogen peroxide, m-chloroperbenzoic acid and peracetic acid.

5. A process according to claim 1 wherein the metal ion is Fe(II) or V(II), and is bound to the solid support by being complexed to a ligand which is attached to the solid support.

6. A process according to claim 5 wherein the ligand is selected from the group consisting of ethylenediamine, 2,2'-bipyridine, 1,10-phananthroline, acetylacetonate, 1,2-Bis(diphenylphosphino)methane, a corrole, a crown ether, a cryptate, cyclopentadienyl, diethylenetriamine, dimethylglyoximate, ethylenediaminetetraacetate, ethlenediaminetriacetate, glycinate, a porphyrin, 2,2',5',2-terpyridine, triazacyclononane, triethylenetetramine, tris(2-aminoethyl)amine and tris (2-diphenylphosphineethyl)amine.

7. A process according to claim 6 wherein the ligand is attached to the solid support via a linker, wherein the linker has the formula: 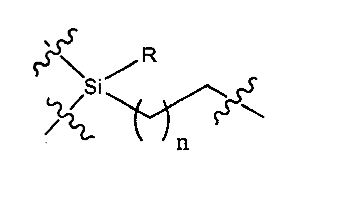 wherein:
R is selected from the group consisting of H, methyl, ethyl, propyl, and OCH₃, OCH₂CH₃;
n is an integer selected from the group consisting of 0, 1, 2, and 3;
and further wherein the Si moiety is attached to the solid support and the CH₂ moiety is attached to the ligand.

8. A process according to any one of claims 1 to 8 wherein the solid support is silica particles.

9. A process according to any one of claims 1 to 8 wherein following the reduction reaction the reaction mixture is filtered to separate the N-demethyl morphinane from the immobilised reducing agent.

10. A process according to any one of claims 1 to 9 wherein the reduction reaction is carried out in an anhydrous alcohol solvent.

11. A process according to any one of the preceding claims wherein the reduction reaction is carried out at a temperature in the range of from 10°C to 120°C.

12. A process according to any one of the preceding claims wherein the reduction reaction is carried out at a temperature in the range of from 40°C to 90°C.

13. A process according to any one of the preceding claims wherein the reduction reaction is carried out for a period of from 15 minutes to 48 hours.

14. A process according to any one of claims 1 to 13 wherein the amount of reducing agent employed is from 0.1 Mole % to 100 Mole %.

15. A process according to any one of claims 1 to 14 wherein the amount of reducing agent employed is from 1 Mole % to 5 Mole %.

## Patentansprüche

1. Verfahren zur Herstellung eines N-Demethylmorphinans, wobei das Verfahren umfasst:
(a) Bereitstellen eines N-Methylmorphinan-N-oxids;
(b) Umsetzen des N-Methylmorphinan-N-oxids mit einem immobilisierten Reduktionsmittel unter reduzierenden Bedingungen in einer Reduktionsreaktion unter Bildung des N-Demethylmorphinans, wobei das immobilisierte Reduktionsmittel ein an einen festen Träger gebundenes Metallion ist.

2. Verfahren gemäß Anspruch 1, wobei das Bereitstellen eines N-Methylmorphinan-N-oxids das Umsetzen eines N-Methylmorphinans mit einem Oxidationsmittel unter oxidierenden Bedingungen in einer Oxidationsreaktion unter Bildung des N-Methylmorphinan-N-oxids umfasst.

3. Verfahren gemäß Anspruch 2, wobei das N-Methylmorphinan aus der Gruppe ausgewählt ist, die aus Morphin, Oripavin, Codein, Thebain und Heroin besteht.

4. Verfahren gemäß Anspruch 2 oder 3, wobei das Oxidationsmittel aus der Gruppe ausgewählt ist, die aus Wasserstoffperoxid, m-Chlorperbenzoesäure und Peressigsäure besteht.

5. Verfahren gemäß Anspruch 1, wobei es sich bei dem Metallion um Fe(II) oder V(II) handelt und es dadurch an den festen Träger gebunden ist, dass es mit einem Liganden, der an den festen Träger gebunden ist, komplexiert ist.

6. Verfahren gemäß Anspruch 5, wobei der Ligand aus der Gruppe ausgewählt ist, die aus Ethylendiamin, 2,2'-Bipyridin, 1,10-Phenanthrolin, Acetylacetonat, 1,2-Bis(diphenylphosphino)methan, einem Corrol, einem Kronenether, einem Kryptat, Cyclopentadienyl, Diethylentriamin, Dimethylglyoximat, Ethylendiamintetraacetat, Ethylendiamintriacetat, Glycinat, einem Porphyrin, 2,2',5',2-Terpyridin, Triazacyclononan, Triethylentetramin, Tris(2-aminoethyl)amin und Tris(2-diphenylphosphinethyl)amin besteht.

7. Verfahren gemäß Anspruch 6, wobei der Ligand über einen Linker an den festen Träger gebunden ist, wobei der Linker die Formel 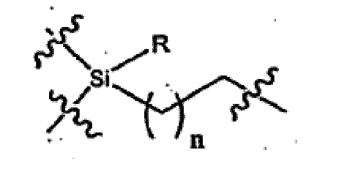 aufweist, wobei:
R aus der Gruppe ausgewählt ist, die aus H, Methyl, Ethyl, Propyl und OCH₃, OCH₂CH₃ besteht;
n eine ganze Zahl ist, die aus der Gruppe ausgewählt ist, die aus 0, 1, 2 und 3 besteht;
und wobei weiterhin die Si-Struktureinheit an den festen Träger gebunden ist und die CH₂-Struktureinheit an den Liganden gebunden ist.

8. Verfahren gemäß einem der Ansprüche 1 bis 8, wobei es sich bei dem festen Träger um Siliciumoxidteilchen handelt.

9. Verfahren gemäß einem der Ansprüche 1 bis 8, wobei das Reaktionsgemisch nach der Reduktionsreaktion filtriert wird, um das N-Demethylmorphinan von dem immobilisierten Reduktionsmittel zu trennen.

10. Verfahren gemäß einem der Ansprüche 1 bis 9, wobei die Reduktionsreaktion in einem wasserfreien Alkohol-Lösungsmittel durchgeführt wird.

11. Verfahren gemäß einem der vorstehenden Ansprüche, wobei die Reduktionsreaktion bei einer Temperatur im Bereich von 10 °C bis 120 °C durchgeführt wird.

12. Verfahren gemäß einem der vorstehenden Ansprüche, wobei die Reduktionsreaktion bei einer Temperatur im Bereich von 40 °C bis 90 °C durchgeführt wird.

13. Verfahren gemäß einem der vorstehenden Ansprüche, wobei die Reduktionsreaktion während einer Zeit von 15 Minuten bis 48 Stunden durchgeführt wird.

14. Verfahren gemäß einem der Ansprüche 1 bis 13, wobei die eingesetzte Menge des Reduktionsmittels 0,1 Mol-% bis 100 Mol-% beträgt.

15. Verfahren gemäß einem der Ansprüche 1 bis 14, wobei die eingesetzte Menge des Reduktionsmittels 1 Mol-% bis 5 Mol-% beträgt.

## Revendications

1. Procédé de préparation d'un N-déméthyl morphinane, le procédé comprenant :
(a) la fourniture d'un N-oxyde de N-méthyl morphinane ;
(b) la réaction du N-oxyde de N-méthyl morphinane avec un agent réducteur immobilisé, dans des conditions réductrices, dans une réaction de réduction pour former le N-déméthyl morphinane, dans laquelle l'agent réducteur immobilisé est un ion métallique lié à un support solide.

2. Procédé selon la revendication 1, dans lequel la fourniture d'un N-oxyde de N-méthyl morphinane comprend la réaction d'un N-méthyl morphinane avec un agent oxydant, dans des conditions oxydantes, dans une réaction d'oxydation pour former le N-oxyde de N-méthyl morphinane.

3. Procédé selon la revendication 2, dans lequel le N-méthyl morphinane est choisi dans le groupe consistant en la morphine, l'oripavine, la codéine, la thébaïne et l'héroïne.

4. Procédé selon la revendication 2 ou 3, dans lequel l'agent oxydant est choisi dans le groupe consistant en le peroxyde d'hydrogène, l'acide m-chloroperbenzoïque et l'acide peracétique.

5. Procédé selon la revendication 1, dans lequel l'ion métallique est Fe(II) ou V(II), et est lié au support solide en étant complexé à un ligand qui est fixé au support solide.

6. Procédé selon la revendication 5, dans lequel le ligand est choisi dans le groupe consistant en l'éthylènediamine, la 2,2'-bipyridine, la 1,10-phénanthroline, l'acétylacétonate, la 1,2-bis(diphényl-phosphino)méthane, un corrole, un éther couronne, un cryptate, le cyclopentadiényle, la diéthylènetriamine, le diméthylglyoximate, l'éthylènediaminetétraacétate, l'éthylènediaminetriacétate, le glycinate, une porphyrine, la 2,2',5',2-terpyridine, le triazacyclononane, la triéthylènetétramine, la tris(2-aminoéthyl)-amine et la tris(2-diphénylphosphineéthyl)amine.

7. Procédé selon la revendication 6, dans lequel le ligand est fixé au support solide via un coupleur, dans lequel le coupleur a la formule : 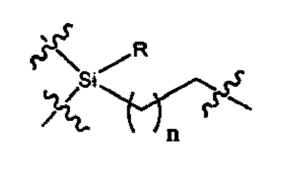 dans laquelle :
R est choisi dans le groupe consistant en H, méthyle, éthyle, propyle, et OCH₃, OCH₂CH₃ ;
n est un entier choisi dans le groupe consistant en 0, 1, 2 et 3 ;
et en outre dans laquelle le fragment Si est fixé au support solide et le fragment CH₂ est fixé au ligand.

8. Procédé selon l'une quelconque des revendications 1 à 7, dans lequel le support solide est des particules de silice.

9. Procédé selon l'une quelconque des revendications 1 à 8, dans lequel à la suite de la réaction de réduction, le mélange réactionnel est filtré pour séparer le N-déméthyl morphinane de l'agent réducteur immobilisé.

10. Procédé selon l'une quelconque des revendications 1 à 9, dans lequel la réaction de réduction est réalisée dans un solvant alcoolique anhydre.

11. Procédé selon l'une quelconque des revendications précédentes, dans lequel la réaction de réduction est réalisée à une température dans la plage de 10 °C à 120 °C.

12. Procédé selon l'une quelconque des revendications précédentes, dans lequel la réaction de réduction est réalisée à une température dans la plage de 40 °C à 90 °C.

13. Procédé selon l'une quelconque des revendications précédentes, dans lequel la réaction de réduction est réalisée pendant une période de 15 minutes à 48 heures.

14. Procédé selon l'une quelconque des revendications 1 à 13, dans lequel la quantité d'agent réducteur employée est de 0,1 % en mole à 100 % en mole.

15. Procédé selon l'une quelconque des revendications 1 à 14, dans lequel la quantité d'agent réducteur employée est de 1 % en mole à 5 % en mole.
